Europäisches Patentamt

European Patent Office

Office européen des brevets

(11) Veröffentlichungsnummer: **0 036 642**
**A2**

(12) # EUROPÄISCHE PATENTANMELDUNG

(21) Anmeldenummer: **81102105.4**

(22) Anmeldetag: **20.03.81**

(51) Int. Cl.³: **A 61 M 25/00**
**// A61M1/03**

(30) Priorität: **21.03.80 DE 3010841**

(43) Veröffentlichungstag der Anmeldung: **30.09.81**
**Patentblatt 81/39**

(84) Benannte Vertragsstaaten: **AT BE CH FR GB IT LI LU NL
SE**

(71) Anmelder: **Uthmann, Ulrich, Dr.-med., Schulstrasse 5,
D-6936 Haag (DE)**

(72) Erfinder: **Uthmann, Ulrich, Dr.-med., Schulstrasse 5,
D-6936 Haag (DE)**

(74) Vertreter: **Geitz, Heinrich, Dr.-Ing.,
Postfach 2708 Kaiserstrasse 156,
D-7500 Karlsruhe 1 (DE)**

(54) **Katheter.**

(57) Der zur Punktion von Blutgefässen, insbesondere
für die Venenpunktion zur Hämodialyse, bestimmte Katheter ist doppelläufig in der Weise ausgebildet, dass
zwei je an eine Schlauchleitung anschliessbare Einzelkatheter (11, 12) einander über eine Längsführung (13)
längsbeweglich zugeordnet sind und mittels letzterer der
eine Einzelkatheter (12) auf den anderen Einzelkatheter
aufschiebbar ist, wobei im Anwendungsfalle die Spitzen
der Einzelkatheter im Blutgefäss eines Probanden in
Strömungsrichtung des Blutes zeigen und die Spitze (20)
des der Blutzufuhr dienenden Einzelkatheters (11) um
ein vorbestimmtes Mass über die Spitze des der Blutentnahme dienenden Einzelkatheters (12) hinausragt, so
dass über den Katheter (11) in ein Blutgefäss zurückgeführtes Blut nicht in den Bereich der Blutentnahme
über den Einzelkatheter (12) gelangt und mithin das Auftreten von Pendelblut ausgeschaltet ist.

ACTORUM AG

EP 0 036 642 A2

811760　　　　　　　　　　　　- 5 -

Anmelder:　Dr. med. Ulrich Uthmann, Schulstraße 5,
　　　　　　6936 Haag / BRD


Katheter
========


Die Erfindung bezieht sich auf einen Katheter zur
Punktion von Blutgefäßen, insbesondere für die Venenpunktion zur Hämodialyse, mit einem durch eine Einstichöffnung in ein Blutgefäß einführbaren Abschnitt
und einer sich daran anschließenden Schlauchleitung.

Katheter dieser Art und für den genannten Zweck sind
in der Praxis bekannt. Es handelt sich dabei in der
Regel um flexible Silikonschläuche mit gegebenenfalls
besonderer Ausbildung der Katheterspitze, also des durch
eine entsprechende Einstichöffnung in ein Blutgefäß
einführbaren Abschnittes. Derartige Katheter sind auch
schon zur Venenpunktion für die venovenöse Hämodialyse verwendet worden. Dabei sind zwei Verfahren bekannt, nämlich die nicht kontinuierliche Dialyse mit mehr oder minder ausgeprägter Hämostase im Dialysefilter und die kontinuierlichen Blutfluß gewährleistende Dialyse mit zwei Punktionsorten und entsprechend zwei Kathetern.

Beide Verfahren haben sich als unzulänglich erwiesen. So treten bei der nicht kontinuierlichen Dialyse große Pendelblutmengen auf, was naturgemäß die Effizienz der Dialyse stark beeinträchtigt. Bei der zweiten Methode, bei der mit zwei Kathetern gearbeitet wird, die an zwei im Abstand voneinander liegenden Punktionsorten in eine Vene eingeführt werden, ist dieser Mangel zwar insofern vermieden, als die Blutzufuhr an dem in Strömungsrichtung vor der Blutentnahmestelle liegenden Punktionsort erfolgt, aber das Erfordernis einer zweimaligen Punktion einer großen Körpervene begründet eine erhöhte Trombose- und Infektionsgefahr.

Durch die Erfindung soll demgegenüber ein Katheter geschaffen werden, mit dem es gelingt, die erhöhte Trombose- und Infektionsgefahr der zweiten Methode und das die erste Methode kennzeichnende Auftreten von Pendelblutmengen zu unterbinden. Diese Aufgabe ist erfindungsgemäß durch die Ausbildung des Katheters als Doppelkatheter gelöst, bei dem die beiden Einzelkatheter mittels
einer Längsführung einander längsbeweglich zugeordnet
sind. Bei der Erfindung handelt es sich mithin um einen
doppelläufigen, in sich verschiebbaren Katheter, mit
dem es gelingt, die im Bereich der Katheterspitzen liegende Eintrittsöffnung des einen Einzelkatheters und die
Austrittsöffnung des anderen Einzelkatheters nach dem
Einführen in ein Blutgefäß, etwa eine Vene, in Strömungsrichtung des Blutes in solchem Abstand voneinander
zu plazieren, daß die Blutzufuhr in Strömungsrichtung
soweit vor der Blutentnahme erfolgt, daß in die Vene
zurückgeführtes Blut nicht in den Bereich der Entnahmestelle gelangt und mithin das Auftreten von Pendelblut
ausgeschaltet ist. Dabei bedarf es lediglich einer Punktion, so daß die durch Mehrfachpunktionen begründeten
Gefährdungen ebenfalls beseitigt sind.

Zweckmäßige Ausgestaltungen sind in den Unteransprüchen
2 bis 11 angegeben.

Bei einer vorteilhaften Ausgestaltung ist die Längsführung eine den einen Einzelkatheter umschließende und

- 8 -

mit diesem verbundene bzw. einstückig ausgeführte Manschette, in welcher der andere Einzelkatheter längsverschieblich und strömungsmitteldicht aufgenommen ist.
Vorteilhaft ist dabei eine Abdichtung am hinteren Ende
der Manschette, wodurch Blutdurchflüsse neben der
Katheterführung verhindert werden. Bei dieser Abdichtung kann es sich um eine die Manschette an dem von den
Katheterspitzen entfernten Ende umschließende Kappe aus
Latex oder einem ähnlichen Material mit je einem Durchgangsloch für jeden Einzelkatheter handeln.

Eine weitere vorteilhafte Ausgestaltung der Erfindung
ist dadurch gekennzeichnet, daß der eine Katheter mit
einer konisch zulaufenden Spitze und mit Perforierungen im Bereich dieser Spitze versehen ist. Dieser Einzelkatheter wird zweckmäßigerweise zur Rückführung von
Blut in das Blutgefäß verwendet.

Eine andere vorteilhafte Ausgestaltung besteht darin,
daß die Spitze des anderen Einzelkatheters, bei dem es
sich zweckmäßigerweise um den der Blutentnahme dienenden Einzelkatheter handelt, mit einer Abschrägung und/
oder seitlichen Löchern versehen ist.

Auch ist es von Vorteil, wenigstens einen der Einzelkatheter mit einer Skalierung und den anderen Einzelkatheter mit einer eine Kontrolle der Lage beider
Katheter zueinander ermöglichenden Markierung auszurüsten und im Bereich der extrakorporalen Enden beider Einzelkatheter Teilstücke aus Latex oder einem ähnlichen Material mit mittels Schlauchklemmen abquetschbaren Strömungswegen vorzusehen.

Schließlich können die genannten Enden der Einzelkatheter auch mittels abstreifbarer Kappen aus Latex oder
einem ähnlichen Material verschlossen sein und die
Böden dieser Kappen können mit einer Wandverdünnung zum
perforierenden Durchführen einer Führungsspirale versehen sein.

Anhand der beigefügten Zeichnung sollen nachstehend eine
Ausführungsform der Erfindung sowie die bestimmungsgemäße
Anwendung des als Ausführungsbeispiel gezeigten Doppelkatheters erläutert werden. In schematischen Ansichten
zeigen:

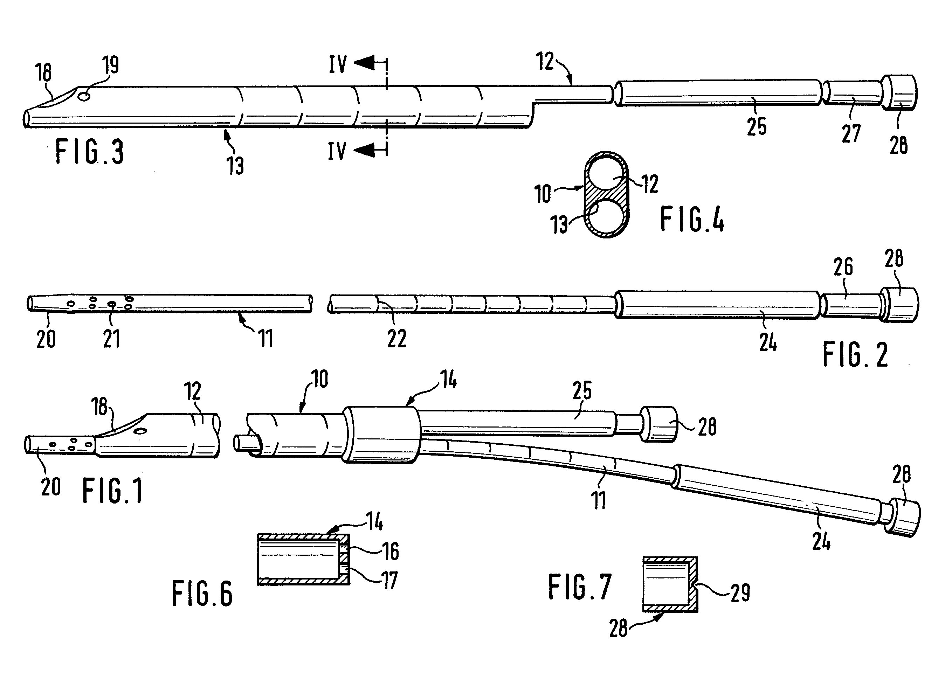

Fig. 1   eine Gesamtansicht eines doppelläufigen, in sich
         verschiebbaren Katheters,

Fig. 2   in einer Ansicht wie in Fig. 1 den der Blutzufuhr dienenden, mit einer Skalierung versehenen Einzelkatheter für sich allein,

Fig. 3   ebenfalls in einer Ansicht wie in Fig. 1 den
der Blutentnahme dienenden Einzelkatheter mit
zugeordneter Längsführung für sich allein,

Fig. 4   einen Querschnitt durch den Bereich der Längsführung des Doppelkatheters gemäß Schnittlinie
IV-IV in Fig. 3,

Fig. 5   die Katheterspitze des Doppelkatheters in einer
perspektivischen Ansicht für sich allein,

Fig. 6   eine der Abdichtung im Bereich des von der
Katheterspitze entfernten Endes der Längsführung dienende Kappe für sich allein in einer
Längsschnittansicht,

Fig. 7   in einer Ansicht wie in Fig. 6 eine weitere
Kappe zum Verschließen der extrakorporalen
Katheterenden und

Fig. 8   in einer Prinzipskizze die bestimmungsgemäße
Anwendung des Doppelkatheters.

Bei dem in seiner Gesamtheit mit 10 bezeichneten Doppelkatheter sind die beiden Einzelkatheter 11, 12 einander
in Richtung ihrer Längserstreckungen verschieblich zugeordnet. Der Katheter 12 ist einstückig mit einer Führungsmanschette 13 ausgebildet, in welcher der andere Katheter 11 aufgenommen ist und die eine Längsführung bildet, so daß die beiden Einzalkatheter 11, 12 in Richtung ihrer Längenerstreckungen gegen-einander verschiebbar, in beliebigen Zuordnungslagen jedoch festlegbar sind. Diese Längsführung ist am hinteren, also von den Katheterspitzen entfernten Ende von einer als Abdichtung gegen den Durchtritt von Blut durch die Katheterführung aufgesetzten Kappe 14 aus Latex oder einem ähnlichen Material umgeben, in deren Rückwand sich zwei Durchgangslöcher 15, 16 befinden, durch die sich die extrakorporalen Enden der Einzelkatheter hindurcherstrecken und dabei dichtend von den Lochrändern umschlossen sind.

Die von der der Abdichtung dienenden Kappe 14 entfernte Spitze des mit der Längsführung 13 ausgerüsteten Einzelkatheters 12 ist mit einer Abschrägung 18 und zwei seitlichen Löchern 19 versehen. Die Spitze 20 des anderen Einzelkatheters 11 ist konisch ausgebildet und besitzt neben einer achsrechten Öffnungen am freien Ende Perforationen 21 in dem konisch zulaufenden Spitzenbereich. Im übrigen ist der Einzelkatheter 11 mit einer Skalierung

- 12 -

22 versehen. Schließlich haben beide Einzelkatheter 11, 12 im Bereich ihrer extrakorporalen Enden je einen Abschnitt 24, 25 aus Latex oder einem ähnlichen Material, die mittels aufsetzbarer Klemmen zusammendrückbar sind, wodurch ein Abquetschen der Strömungswege gelingt. An diese Abschnitte schließen sich Teilstücke 26, 27 an, die in hier nicht weiter interessierender Weise mit der Blutab- bzw. -zufuhr dienenden Schlauchleitungen verbindbar und im übrigen mittels abstreifbarer Kappen 28 mit einer Wandverdünnung 29 im Kappenboden verschlossen sind.

Bei der Handhabung des erfindungsgemäßen Doppelkatheters 10 wird mittels eines Skalpells über einer zu punktierenden Vene eine Hautinzision entsprechender Größe gelegt und durch diese in bekannter Weise die Vene mittels einer Punktionsnadel punktiert. Danach ist, wie gleichfalls bekannt, eine Führungsspirale auf entsprechender Länge in die punktierte Vene einzubringen und anschließend über letztere der Einzelkatheter 11 in die Vene einzuführen, wobei erforderlichenfalls der Boden der Kappe 28 im Bereich der Wandverdünnung 29 durchstoßen wird. Danach wird der zweite Einzelkatheter 12 mit der Längsführung 13 über den in die Vene eingebrachten Einzelkatheter 11 aufgeschoben, bis eine den Erfordernissen entsprechende Einschieblänge vorliegt, wobei die Venenpunktionsstelle entsprechend der Stärke der Längsführung aufgeweitet wird. Nunmehr kann die dem Einführen des ersten Katheters 11 dienende Führungsspirale zurückgezogen, der zweite Katheter 12 mit der Längsführung 13 mittels einer Naht fixiert und anschliessend der erste Katheter 11 den Erfordernissen entsprechend weiter in die Vene eingebracht werden. Damit ist das Einbringen des Doppelkatheters in eine Vene beendet und die Einzelkatheter sind in bekannter, nicht weiter interessierender Weise über Schlauchleitungen an ein Dialysesystem anschließbar.

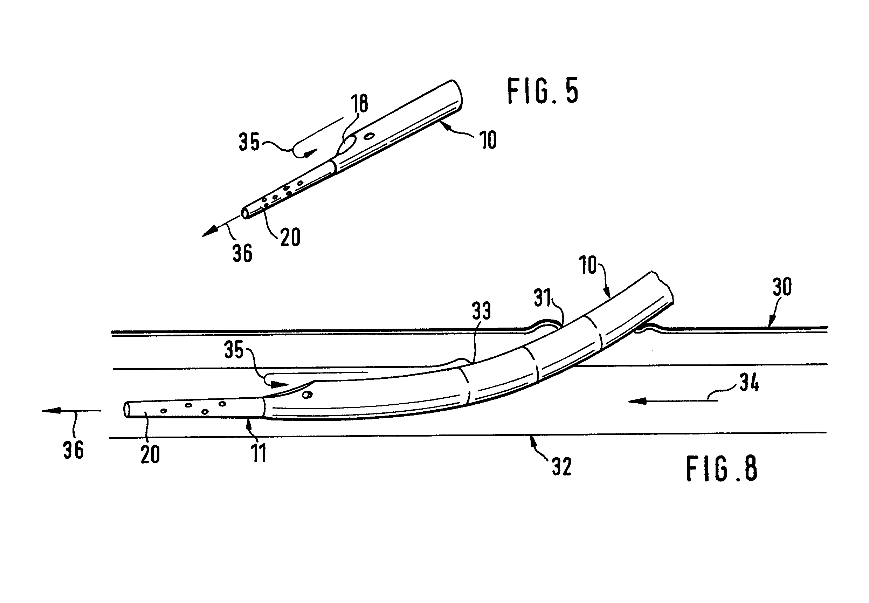

Fig. 8 veranschaulicht schematisch die Anwendung des erfindungsgemäßen Doppelkatheters. Die Haut 30 ist bei 31 über einer Körpervene 32 mittels eines Skalpells mit einer Hautinzision entsprechender Größe versehen und durch letztere ist mittels einer Punktionsnadel bei 33 die Vene punktiert. In der oben erläuterten Weise wurde sodann eine Führungsspirale durch die Hautinzision 31 und die Punktionsstelle 33 eingeführt und nach dem Entfernen der Punktionsnadel über diese Führungsspirale der Einzelkatheter 11 in die Vene eingebracht. Anschliessend erfolgte das Aufschieben des zweiten Katheters 12 mittels der Längsführung 13 auf dem ersten Katheter 11, wobei die Punktionsstelle 33 entsprechend dem Querschnitt der Längsführung aufgeweitet wurde. Anschließend ist die Führungsspirale entfernt und der zweite Einzelkatheter mit der Längsführung 13 in nicht weiter dargestellter Weise in der in der Zeichnung veranschaulichten Lage fixiert worden. Danach wurde der als Führung beim Aufschieben des zweiten Einzelkatheters 12 dienende Einzelkatheter 11 in der Längsführung und mithin mit der Katheterspitze 20 innerhalb der Vene in der durch den Pfeil 34 angedeuteten Strömungsrichtung des in der Vene geführten Blutes vorgeschoben, so daß die Spitze 20 des Einzelkatheters 11 in Strömungsrichtung des Blutes vor der mit einer Anschrägung 18 versehenen Spitze des zweiten Einzelkatheters 12 liegt. Der mit der Längsführung 13 ausgerüstete Katheter 12 dient der Blutentnahme. Die Blutentnahme erfolgt in Strömungsrichtung gemäß Pfeil 34 gesehen, vor der Spitze 20 des Einzelkatheters 12, wie der Umkehrpfeil 35 andeutet, wonach das Blut in hier nicht weiter interessierender Weise zu einem Dialysesystem geführt wird. Das von diesem System zurückfließende Blut wird über den Einzelkatheter 11 wieder in die Vene eingeführt und tritt im Bereich der konischen Spitze 20 durch die achsrechte Austrittsöffnung gemäß Pfeil 36 sowie über die Perforationen im konischen Spitzenbereich dieses Katheters in die Vene ein. Angesichts der in Strömungsrichtung des Blutes in der Vene in erheblichem Abstand vor der Blutentnahmestelle liegenden Austrittsöffnung des Einzelkatheters 11 ist eine Vermischung des vom Dialysesystem zurückgeführten Blutes mit dem über den Einzelkatheter 12 entnommenen Blut wirksam unterbunden.

DR. ING. HEINRICH GEITZ PATENTANWALT 7500 KARLSRUHE 1, POSTFACH 2708

0036642

811760

Anmelder:    Dr. med. Ulrich Uthmann
             Schulstraße 5

             6936  Haag / BRD


Patentansprüche
================


1. Katheter zur Punktion von Blutgefäßen, insbesondere für die Venenpunktion zur Hämodialyse, mit einem durch eine Einstichöffnung in ein Blutgefäß einführbaren Abschnitt und einer sich daran anschließenden Schlauchleitung, gekennzeichnet durch die Ausbildung als Doppelkatheter (10), bei dem die beiden Einzelkatheter (11, 12) mittels einer Längsführung (13) einander längsbeweglich zugeordnet sind.


2. Doppelkatheter nach Anspruch 1, dadurch gekennzeichnet, daß die Längsführung eine den einen Katheter (12)

- 2 -

811760

umschließende und mit letzterem verbundene Manschette (13) ist, in welcher der andere Katheter (11) längsverschieblich aufgenommen ist.

3. Doppelkatheter nach Anspruch 2, gekennzeichnet durch die einstückige Ausbildung der Längsführung (13) mit dem einen Katheter (12).

4. Doppelkatheter nach Anspruch 2 oder 3, gekennzeichnet durch eine Blutdurchflüsse neben der Katheterführung in der Manschette (13) verhindernde Abdichtung (14) an dem von den Katheterspitzen entfernten Manschettenende.

5. Doppelkatheter nach Anspruch 4, dadurch gekennzeichnet, daß die Abdichtung eine die Manschette (13) umschließende Kappe (14) aus Latex oder einem ähnlichen Material mit je einem Durchgangsloch (15, 16) für jeden Einzelkatheter ist.

6. Doppelkatheter nach einem der Ansprüche 1 bis 5, dadurch gekennzeichnet, daß der eine Katheter (11) mit einer konisch zulaufenden Spitze (20) und mit Perforierungen (21) im Bereich dieser Spitze versehen ist.

7. Doppelkatheter nach einem der Ansprüche 1 bis 6, dadurch gekennzeichnet, daß die Spitze des einen Katheters (12) mit einer Abschrägung (18) und/oder seitlichen Löchern (19) versehen ist.

8. Doppelkatheter nach einem der Ansprüche 1 bis 7, dadurch gekennzeichnet, daß wenigstens einer der Einzelkatheter (11, 12) mit einer Skalierung (22) und der andere Einzelkatheter mit einer eine Kontrolle der Lage beider Katheter zueinander ermöglichenden Markierung versehen ist.

9. Doppelkatheter nach einem der Ansprüche 1 bis 8, dadurch gekennzeichnet, daß die Einzelkatheter (11, 12) im Bereich ihrer extrakorporalen Enden mit Teilstücken (24, 25) aus Latex oder einem ähnlichen Material versehen und daß die Querschnitte im Bereich dieser Teilstücke mittels aufsetzbarer Klemmen abquetschbar sind.

10. Doppelkatheter nach einem der Ansprüche 1 bis 9, dadurch gekennzeichnet, daß die extrakorporalen Enden der Einzelkatheter (11, 12) mittels abstreifbarer Kappen (28) aus Latex oder einem ähnlichen Material verschlossen sind.

811760           - 4 -

11. Doppelkatheter nach Anspruch 10, dadurch gekennzeichnet, daß die Böden der Kappen (28) mit einer Wandverdünnung (29) zum perforierenden Durchführen einer Führungsspirale versehen sind.

- 5 -

FIG.3

18  19  IV  12

13  IV  25  27  28

10  12
13  FIG.4

26  28

20  21  11  22  24  FIG.2

10  14  25  28

18  12

20  FIG.1  11  24  28

14

16

17

FIG.6  FIG.7  29

28

1/2

0036642

FIG.5

FIG.8